# FASCICULE DE BREVET EUROPEEN

(11) **EP 1 774 917 B1**
(45) Date de publication et mention de la délivrance du brevet: **17.02.2010**
(21) Numéro de dépôt: 05109502.4
(22) Date de dépôt: 13.10.2005
(51) Int. Cl.: A61B 17/28, A61B 19/00

(54) **Instrument médical et son procédé d'identification**
Medizinisches Instrument und Verfahren zu dessen Erkennung
Medical instrument and its identification procedure

(43) Date de publication de la demande: 18.04.2007
(73) Titulaire: MBBS SA, 2016 Cortaillod (CH)
(72) Inventeur: Bui, Ngoc Chau, 2046, Fontaines (CH); Gaisch, Robert, 2036, Cormondrèche (CH)
(74) Mandataire: GLN

(56) Documents cités:
- EP-A- 1 155 654
- DE-A1- 10 014 542
- DE-U1- 20 012 237
- US-A1- 2002 161 460

## Description

L'invention appartient au domaine de l'instrumentation médicale. Elle concerne plus précisément un instrument médical dont un élément actif est muni d'un transpondeur. L'invention concerne également un procédé de lecture/écriture de ce transpondeur.

Les instruments médicaux et, en particulier, les instruments chirurgicaux, font généralement l'objet d'un suivi rigoureux destiné à garantir leurs conditions d'utilisation et la sécurité des patients. Ce suivi consiste à enregistrer et mettre à jour toute information utile portant sur l'instrument, par exemple, son numéro de série, les dates de ses opérations de maintenance ou de stérilisation, le nombre d'utilisations etc... A cet effet, les étiquettes électroniques, encore appelées transpondeurs ou TAGs RFID, sont largement utilisées, car elles présentent des avantages considérables. Solidaires d'un instrument, elles permettent, en association avec un module lecture / écriture doté d'une antenne et un ordinateur central, l'écriture, la lecture et le stockage d'un grand nombre de données relatives à cet instrument. L'enregistrement d'une information peut être réalisé automatiquement, lors d'une étape de nettoyage, par exemple, de façon à limiter l'erreur d'origine humaine. Un tel mode de fonctionnement est décrit, par exemple, dans le document EP 0992212. Les étiquettes électroniques sont, en outre, extrêmement fiables et supportent les températures élevées des procédés de stérilisation.

Concernant les instruments métalliques simples, tels que des ciseaux, des scalpels, etc, les transpondeurs sont généralement fixés sur une zone non fonctionnelle de l'outil, par exemple, son manche. Ils sont alors disposés dans une boîte métallique, elle même soudée au manche.

Pour les instruments plus complexes, comportant plusieurs pièces montées ensemble, dont au moins un outil, le transpondeur est généralement solidaire d'une pièce extérieure, de façon à être facilement accessible en lecture et en écriture. Le document EP 1480153 montre, par exemple, un endoscope formé d'une partie invasive, d'une unité de couplage optique, et d'une commande munie d'un transpondeur. Cette disposition du transpondeur, sur une pièce externe de l'endoscope, n'est pas idéale, particulièrement en cas de démontage partiel ou total de l'instrument, pour un nettoyage, par exemple, ou pour le changement d'un élément. En effet, une fois l'instrument démonté, les différentes pièces et, en particulier, les pièces actives, ne sont plus identifiables. Si plusieurs instruments sont démontés simultanément, leurs pièces peuvent être interverties au remontage. Par conséquent, seule la traçabilité de la pièce étiquetée peut être réellement garantie. Plus grave même, une incompatibilité entre les différentes pièces peut survenir suite à une telle interversion. Le fonctionnement de l'instrument et, par conséquent, la santé du patient, sont alors hypothéqués.

Le document US-A- 2002/0161460 décrit un instrument médical comportant un élément tubulaire, un outil, des moyens d'actionnement et un transpondeur passif.

L'invention permet de pallier ces inconvénients pour un certain type d'instrument médical, tel qu'un laparoscope, comportant un outil interchangeable solidaire d'une tige montée à l'intérieur d'un actionneur.

Plus précisément, l'invention concerne un instrument médical comportant un élément tubulaire métallique, un outil fixé à l'une de ses extrémités et des moyens d'actionnement de l'outil couplés à son autre extrémité. Selon l'invention :
- l'élément tubulaire est monté coulissant à l'intérieur d'une gaine, son coulissement, sous l'effet des moyens d'actionnement, permettant de faire fonctionner l'outil, et renferme, de manière étanche, un transpondeur passif cylindrique disposé selon son axe et contenant des informations relatives à l'outil et à sa compatibilité avec l'instrument lui-même, et
- la gaine est muni d'un deuxième transpondeur passif contenant des informations relatives à l'instrument lui-même et à sa compatibilité avec l'élément tubulaire.

Les transpondeurs passifs cylindriques sont bien connus de l'homme de métier. Ils sont employés pour le marquage d'objets divers, et sont généralement logés dans des cavités dont l'axe longitudinal est parallèle ou perpendiculaire à la surface de l'objet. La lecture/écriture de ces TAGs se fait à l'aide d'une antenne cylindrique disposée, selon le cas, au sommet du transpondeur ou dans son voisinage immédiat. Ce procédé de lecture/écriture est peu efficace car il ne permet de récolter qu'une faible partie du signal émis par le transpondeur.

L'invention propose, outre un instrument médical muni d'un TAG, un procédé de lecture/écriture de ce TAG, plus performant que les procédés couramment employés.

Plus précisément, l'invention concerne un procédé d'identification et de suivi de l'instrument médical défini ci-dessus, consistant à :
- se doter d'un module lecture/écriture apte à générer et traiter un signal RF contenant de l'information, d'une antenne formée d'un bobinage annulaire apte à émettre et recevoir le signal RF, et d'un ordinateur central destiné à stocker et traiter l'information issue du signal RF, et
- disposer le bobinage autour de l'élément tubulaire, et le déplacer, si nécessaire, le long de l'élément tubulaire jusqu'à capter le signal issu du transpondeur passif.

L'invention sera mieux comprise à la lecture de la description qui suit, faite en référence au dessin annexé, dans lequel :
- la figure 1 est une vue schématique de côté d'un laparoscope selon l'invention,
- les figures 2, 3 et 4 sont des vues agrandies de trois modes de réalisation d'un élément de cet instrument, et
- la figure 5 représente une étape du procédé d'identification et de suivi du laparoscope selon l'invention.

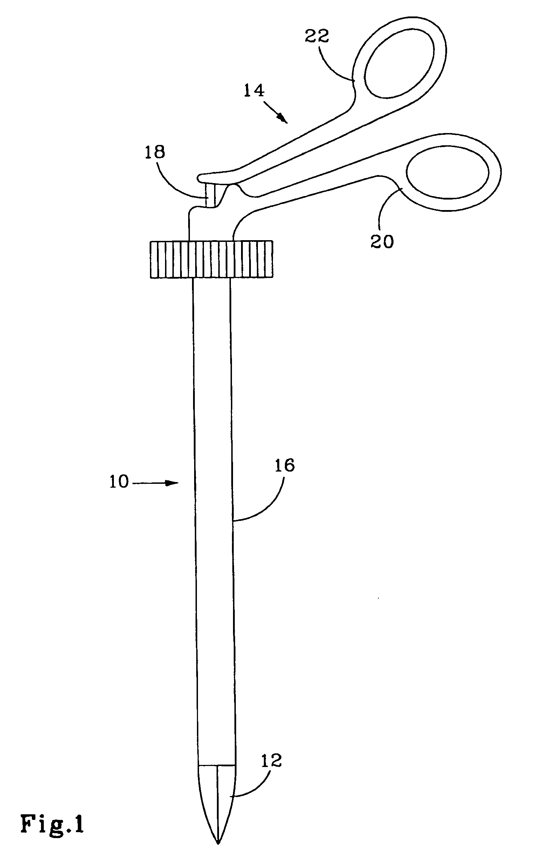

Le laparoscope représenté sur les figures 1 à 4 comporte, classiquement, un actionneur 10 muni d'un outil 12 tel qu'une pince, et une poignée 14. L'actionneur 10 est formé d'une gaine rigide 16, et d'une tige 18 montée coulissante à l'intérieur de celle-ci et solidaire de l'outil 12 par l'une de ses extrémités. La poignée 14 est constituée d'une partie fixe 20 par rapport à l'actionneur 10 et d'un levier mobile 22. La tige 18 est fixée au levier mobile 22 par son extrémité opposée à l'outil 12. Lorsque le levier 22 bascule sous l'action de l'utilisateur, la tige 18 coulisse à l'intérieur de la gaine 16. L'outil 12 est articulé et coopère avec la gaine 16 de sorte que le coulissement de la tige 18 entraîne son ouverture et sa fermeture. Ce mode de fonctionnement du laparoscope est bien connu de l'homme de métier et ne sera donc pas décrit plus longuement.

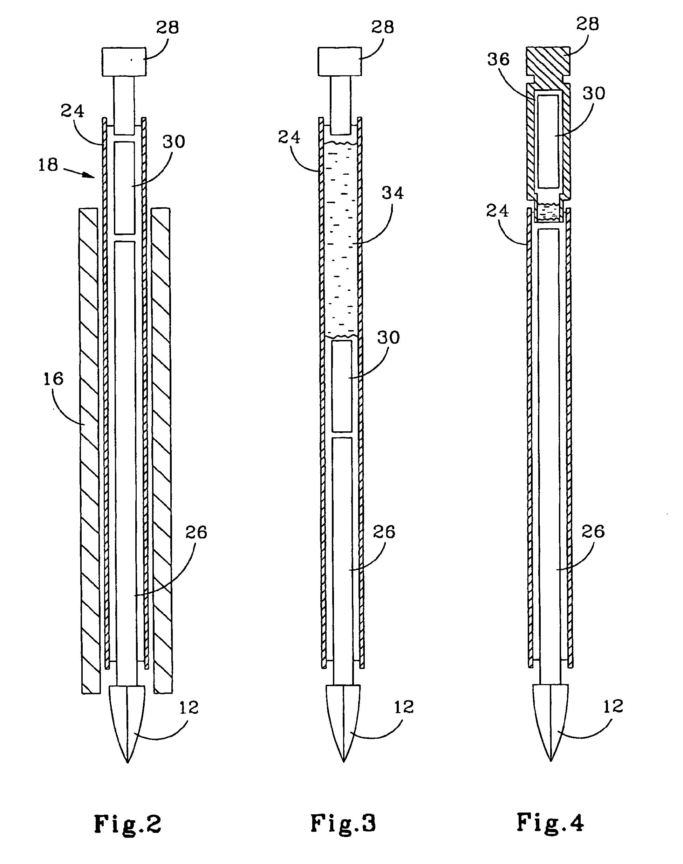

L'actionneur 10 du laparoscope de la figure 1 est représenté en coupe en figure 2. La tige 18 comporte un tube 24 de diamètre extérieur légèrement inférieur au diamètre intérieur de la gaine 16, de sorte que le guidage de la tige 18 par la gaine 16 est optimal. Une tige pleine 26 à l'extrémité de laquelle est monté l'outil 12, est engagée, jusqu'à l'outil 12, dans le tube 24 et soudée à celui-ci de manière étanche. Un clou 28 est engagé dans le tube 24 à l'autre extrémité et soudé à celui-ci de manière également étanche. Le clou 28 est fixé, par sa tête, au levier 22 par des moyens de fixation non représentés.

Selon l'invention, un transpondeur passif cylindrique 30 est inséré, à l'intérieur du tube 24, dans un logement laissé libre à l'extrémité du tube 24, entre la tige pleine 26 et le clou 28. Ce transpondeur 30 est du type de celui décrit dans le brevet US 5572410. Il comporte, classiquement, un circuit électronique muni de deux plages de contact et une bobine formée d'un fil enroulé sur un noyau en ferrite et dont les deux extrémités sont soudées aux plages de contact. Il peut être coulé dans de la résine selon un procédé bien connu de l'homme de métier.

Le montage du transpondeur 30 à l'intérieur du tube 24 étant étanche, le tranpondeur 30 est à l'abri de toute contrainte mécanique ou chimique, ce qui permet d'éviter de l'encapsuler dans un boîtier avant sa mise en place. L'économie et la simplification qui en découlent sont considérables.

Le transpondeur 30 contient un grand nombre d'informations relatives à l'outil 12 et à sa compatibilité avec le laparoscope auquel il appartient. A titre d'exemple, les données suivantes peuvent être stockées : numéro de série, nom du fabricant, nom de l'assembleur, dates des utilisations, des maintenances etc.... En outre, un code de compatibilité peut être introduit entre l'outil 12 et le laparoscope. Pour cela, il est nécessaire que le laparoscope comporte lui-même un transpondeur fixé, par exemple, à sa poignée 14 et porteur d'un code de compatibilité identique.

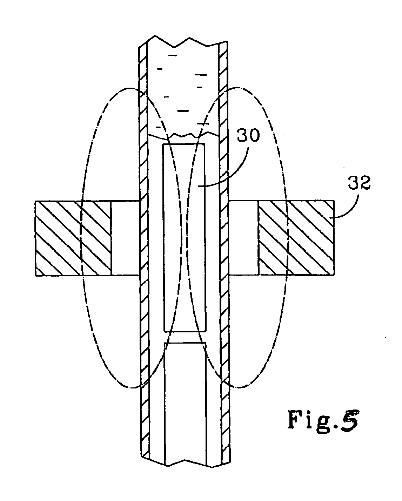

La lecture et l'écriture d'informations au niveau du transpondeur 30, se font à l'aide d'un module lecture/écriture, par l'intermédiaire d'une antenne annulaire 32 représentée en coupe à la figure 5. L'antenne 32, formée d'une bobine de fil en anneau, est disposée autour de la tige 18 et déplacée le long de celle-ci jusqu'à recevoir le signal du transpondeur 30. Ce signal est maximum lorsque l'antenne 32 est située à hauteur du transpondeur 30. En effet, les lignes de champ du transpondeur cylindrique 30 passent par son noyau de ferrite et se referment à l'extérieur de la tige 18, formant un volume sensiblement torique. Lorsque l'antenne 32 est disposée en face du transpondeur 30, les lignes de champ traversent l'antenne 32 sur toute sa périphérie, ce qui permet de capter un maximum de signal.

Ce procédé de lecture/écriture est particulièrement avantageux par rapport aux procédés communément employés pour un transpondeur cylindrique. Ceux-ci consistent généralement à placer une antenne, elle-même cylindrique, au sommet du transpondeur, ou à son voisinage immédiat, et ne permettent de capter d'une faible partie du signal. La sensibilité accrue de la lecture/écriture selon l'invention permet, par exemple, d'augmenter l'épaisseur des parois métalliques, tout en conservant un signal suffisant.

En outre, grâce au procédé de lecture/écriture décrit, il est possible de disposer le transpondeur 30 en un emplacement quelconque à l'intérieur du tube 24. On a représenté partiellement, par exemple, sur la figure 3, un laparoscope qui diffère du laparoscope de la figure 2 en ce que le transpondeur 30 est logé au centre du tube 24. La tige 26 est, à cet effet, plus courte qu'en figure 2. De plus, de la résine 34 a été injectée dans le tube 24 après insertion du transpondeur 30, afin de l'immobiliser. Cette configuration du laparoscope ne modifie en rien la fonction ni le mode de lecture/écriture du transpondeur 30.

Un autre mode de réalisation d'un laparoscope selon l'invention est illustré en figure 4. Dans ce mode de réalisation, la tige 26 occupe pratiquement tout l'espace à l'intérieur du tube 24. Le clou 28 est creux de telle sorte qu'il forme un logement 36 dans lequel est disposé le transpondeur 30. L'extrémité du clou est fermée à l'aide de résine, afin de bloquer le transpondeur 30 dans son logement 36. Comme pour les exemples précédents, le clou 28 est soudé au tube 24 de manière étanche.

Ainsi a été décrit un instrument médical comportant un outil équipé d'un transpondeur cylindrique permettant son identification et sa traçabilité individuelles, indépendamment de celles de l'instrument.

## Revendications

1. Instrument médical comportant un élément tubulaire métallique (24, 42), un outil (12, 38) fixé à l'une de ses extrémités et des moyens d'actionnement (22) de l'outil (12) couplés à son autre extrémité, dans lequel
- ledit élément tubulaire (24, 42) est monté coulissant à l'intérieur d'une gaine (16), son coulissement, sous l'effet desdits moyens d'actionnement (22), permettant de faire fonctionner l'outil (12), et renferme, de manière étanche, un premier transpondeur passif cylindrique (30) disposé selon son axe et contenant des informations relatives à l'outil (12, 38) et à sa compatibilité avec l'instrument lui-même, et
- la gaine (16) est munie d'un deuxième transpondeur passif contenant des informations relatives à l'instrument lui-même et à sa compatibilité avec l'élément tubulaire (24,42).

2. Procédé d'identification et de suivi d'un instrument médical selon la revendication 1, **caractérisé en ce qu'**il consiste à :
- se doter d'un module lecture / écriture apte à générer et traiter un signal RF contenant de l'information, d'une antenne (32) formée d'un bobinage annulaire apte à émettre et recevoir ledit signal RF, et d'un ordinateur central destiné à stocker et traiter l'information issue dudit signal RF, et
- disposer l'antenne annulaire autour de l'élément tubulaire (24) et la déplacer, si nécessaire, le long dudit élément tubulaire (24) jusqu'à capter le signal issu du transpondeur passif (30).

## Claims

1. A medical instrument including a metal tubular member (24, 42), a tool (12, 38) attached to one of its ends and means (22) for actuating the tool (12), coupled to its other end, wherein
- said tubular member (24, 42) is slidably mounted inside a sheath (16), its sliding under the effect of said actuation means (22) makes it possible to operate the tool (12), and sealably contains a first cylindrical passive transponder (30) positioned along its axis and containing information relating to the tool (12, 38) and to its compatibility with the instrument itself, and
- the sheath (16) is provided with a second passive transponder containing information relating to the instrument itself and to its compatibility with the tubular member (24, 42).

2. A method for identification and follow-up of a medical instrument according to claim 1, **characterized in that** it consists of:
- providing itself with a read/write module capable of generating and processing an RF signal containing information, with an antenna (32) formed with an annular coil capable of transmitting and receiving said RF signal, and with a central computer intended to store and process the information from said signal, and
- arranging the annular antenna around the tubular member (24) and displacing it, if necessary, along said tubular member (24) until it picks up the signal from the passive transponder (30).

## Patentansprüche

1. Medizinisches Instrument, umfassend ein metallisches röhrenförmiges Element (24, 42), ein Werkzeug (12, 38), das an einem seiner Enden befestigt ist, und Mittel zum Betätigen (22) des Werkzeugs (12), die mit seinem anderen Ende gekoppelt sind, wobei
- das röhrenförmige Element (24, 42) im Innern einer Ummantelung (16) verschiebbar gelagert ist, wobei das Verschieben desselben unter Einwirkung der Betätigungsmittel (22) die Bedienung des Geräts (12) ermöglicht, und abdichtend einen ersten zylindrischen passiven Transponder (30) einschließt, der an der Achse desselben entlang angeordnet ist und Informationen über das Werkzeug (12, 38) und seine Kompatibilität mit dem Werkzeug selber enthält, und
- die Ummantelung (16) mit einem zweiten passiven Transponder versehen ist, der Informationen über das Instrument selber und seine Kompatibilität mit dem röhrenförmigen Element (24, 42) enthält.

2. Verfahren zum Identifizieren und Verfolgen eines medizinischen Instruments nach Anspruch 1, **dadurch gekennzeichnet, dass** es aus folgenden Schritten besteht:
- Bereitstellen eines Lese-/Schreib-Moduls, das dazu geeignet ist, ein Informationen enthaltendes HF-Signal zu erzeugen und zu verarbeiten, einer Antenne (32), die aus einer ringförmigen Wicklung gebildet ist, die in der Lage ist, das HF-Signal zu senden und zu empfangen, und eines Zentralrechners, der dazu gedacht ist, die aus dem HF-Signal stammenden Informationen zu speichern und zu verarbeiten, und
- Anordnen der ringförmigen Antenne um das röhrenförmige Element (24) herum und gegebenenfalls Verlagern derselben an dem röhrenförmigen Element (24) entlang, bis das aus dem passiven Transponder (30) stammende Signal empfangen wird.
